# EUROPÄISCHE PATENTANMELDUNG

(11) **EP 3 064 250 A1**
(43) Veröffentlichungstag der Anmeldung: **07.09.2016**
(21) Anmeldenummer: 15158064.4
(22) Anmeldetag: 06.03.2015
(51) Int. Cl.: A61M 37/00, A61B 17/20, A61D 7/00

(54) **Hautstechwerkzeug und Stechwerkzeug zum lokalen Aufstechen einer menschlichen oder tierischen Haut und Stechwerkzeuganordnung**

(71) Anmelder: MT.DERM GmbH, 14167 Berlin (DE)
(72) Erfinder: Scherkowski, Dirk, 12489 Berlin (DE)
(74) Vertreter: Bittner, Thomas L.

(57) **Zusammenfassung**

Hautstechwerkzeug zum lokalen Aufstechen einer menschlichen oder einer tierischen Haut mit einem proximalen und einem distalen Werkzeugabschnitt, in welchem eine Stechspitze angeordnet ist, einer oder mehreren Nadeln (1, 2), bei denen ein Nadelschaft (1c, 2c) im Bereich des proximalen Werkzeugabschnitts und eine Nadelspitze als Teil der Stechspitze (3) im Bereich des distalen Werkzeugabschnitts angeordnet sind, und einem Zuführreservoir (4), welches sich als offenes Reservoir zwischen einander gegenüberliegenden Nadelabschnitten (1a, 1b, 2a, 2b) der einen oder der mehreren Nadeln und in Richtung des proximalen Werkzeugabschnitts hinter der Stechspitze erstreckt, wobei das Zuführreservoir in Fließverbindung mit der Stechspitze steht, derart, dass im Betrieb eine einzutragende Substanz aus dem Zuführreservoir fließend zur Stechspitze hin gelangen kann.

## Beschreibung

Die Erfindung betrifft ein Hautstechwerkzeug zum lokalen Aufstechen einer menschlichen oder einer tierischen Haut sowie eine Stechwerkzeuganordnung.

### Hintergrund

Hautstechwerkzeuge werden genutzt, um eine menschliche oder eine tierische Haut lokal aufzustechen, zum Beispiel zum Einbringen einer Substanz in die Haut. Sofern eine Substanz eingetragen werden soll, kann es sich hierbei beispielsweise um eine Farbe, eine medizinische Substanz und / oder eine kosmetische Substanz handeln. Im Fall der Farbe kann diese dazu dienen, ein Tattoo oder permanentes Make-up zu applizieren.

Hautstechwerkzeuge können mit einer oder mehreren Nadeln gebildet sein. Im Fall mehrerer Nadeln kann eine Nadelgruppe gebildet sein. So offenbart das Dokument US 2012 / 0029549 A1 eine Tattoo-Nadelvorrichtung, bei der mehrere Einzelnadeln zu einer Gruppe von Nadeln zusammengefügt sind, wobei Nadelschäfte miteinander verlötet sind. Von der Längsrichtung der jeweiligen Nadel im Bereich des Nadelschafts ausgehend sind die Nadeln im Bereich mit der Nadelspitze nach innen gebogen, um Stechspitzen der Nadeln nebeneinander liegend anzuordnen.

Auch im Dokument JP 2000342332 A sind Hautstechwerkzeuge beschrieben, bei denen mehrere Nadeln zu einer Nadelgruppe zusammengefasst sind. Weitere Stechwerkzeuge mit mehreren Nadeln sind im Dokument CN 201862140 U beschrieben. Einzelnadeln und Stechvorrichtungen, bei denen mehrere Nadeln im Bereich einer Applikationsfläche angeordnet sind (Nadelplatte), sind im Dokument US 2011 / 0042847 A1 offenbart.

Weitere Nadeln zum Aufstechen einer Haut sind beschrieben im Dokument US 2005 / 0066775 A1.

### Zusammenfassung

Aufgabe der Erfindung ist es, ein Hautstechwerkzeug zum lokalen Aufstechen einer menschlichen oder einer tierischen Haut sowie eine Stechwerkzeuganordnung mit mehreren Hautstechwerkzeugen anzugeben, die ein effizienteres lokales Aufstechen der Haut ermöglichen.

Zur Lösung der Aufgabe ist ein Hautstechwerkzeug zum lokalen Aufstechen einer menschlichen oder einer tierischen Haut nach Anspruch 1 geschaffen. Weiterhin ist eine Stechwerkzeuganordnung mit mehreren Hautstechwerkzeugen nach dem unabhängigen Anspruch 14 vorgesehen. Ausgestaltungen sind Gegenstand von abhängigen Ansprüchen.

Nach einem Aspekt ist ein Hautstechwerkzeug zum lokalen Aufstechen einer menschlichen oder einer tierischen Haut geschaffen. Das Hautstechwerkzeug weist einen proximalen Werkzeugabschnitt und einen distalen Werkzeugabschnitt auf, in welchem eine Stechspitze angeordnet ist, die beim Einsatz des Hautstechwerkzeugs auf oder gegen die Hautoberfläche angewendet wird. Ein oder mehrere Nadeln sind vorgesehen, bei denen ein Nadelschaft im Bereich des proximalen Werkzeugabschnitts und eine Nadelspitze als Teil der Stechspitze im Bereich des distalen Werkzeugabschnitts angeordnet sind. Des Weiteren ist ein Zuführreservoir gebildet, welches sich als offenes Reservoir zwischen einander gegenüberliegenden Nadelabschnitten der einen oder der mehreren Nadeln und in Richtung des proximalen Werkzeugabschnitts hinter der Stechspitze erstreckt. Das Zuführreservoir steht in Fließverbindung mit der Stechspitze, derart, dass im Betrieb eine einzutragende Substanz aus dem Zuführreservoir fließend zur Stechspitze hin gelangen kann. Die einander gegenüberliegenden Nadelabschnitte an der einen Nadel oder an einer der mehreren Nadeln, benachbart zu dem Zuführreservoir, weisen einen Nadelabschnitt, welcher von der Nadellängsachse weg geneigt oder gebogen ist, und einen weiteren Nadelabschnitt auf, welcher zwischen dem Nadelabschnitt und der Nadelspitze angeordnet und zur Nadellängsachse hin geneigt oder gebogen ist.

Nach einem weiteren Aspekt ist eine Stechwerkzeuganordnung mit mehreren Hautstechwerkzeugen zum lokalen Aufstechen einer menschlichen oder einer tierischen Haut geschaffen, wobei die mehreren Hautstechwerkzeuge an einer gemeinsamen Werkzeugaufnahme angeordnet sind.

Bei dem Hautstechwerkzeug sind der von der Nadellängsachse weg geneigte oder gebogene Nadelabschnitt sowie der weitere, zur Nadellängsachse hin geneigte oder gebogene Nadelabschnitt benachbart zum Zuführreservoir angeordnet und insoweit das offene Reservoir bildend oder begrenzend gebildet.

Die geneigten Nadelabschnitte können gerade ausgeführt sein.

Verschiedene Typen von Nadeln können zur Anwendung kommen, wozu zum Beispiel Flachnadeln, die beispielweise aus einem Blech gestanzt sein können, sowie Voll- und Hohlnadeln gehören, die einen runden, einen ovalen oder einen eckigen Querschnitt aufweisen können. Auch kann eine einzelne Nadel in verschiedenen Abschnitten in Längsrichtung unterschiedliche Querschnittsformen aufweisen.

Das Haustechwerkzeug oder die Stechwerkzeuganordnung mit mehreren Hautstechwerkzeugen können lösbar oder nicht lösbar an einem Handgerät zum lokalen Aufstechen einer menschlichen oder einer tierischen Haut aufgenommen sein. In einer Ausgestaltung weist das Handgerät eine Antriebseinrichtung auf, mit der das Hautstechwerkzeug oder die Stechwerkzeuganordnung im Betrieb vor- und zurückbewegt wird. In einer Ausgestaltung wird mittels der Antriebseinrichtung eine Antriebskraft zum Bewegen des Hautstechwerkzeugs oder der Stechwerkzeuganordnung mittels Umwandlung elektrischer Energie erzeugt. Derartig Handgeräte sind als solche in verschiedenen Ausführungsformen bekannt, insbesondere mit nicht manuellem Antrieb.

Die Stechspitze kann mit mehreren Nadelspitzen gebildet sein. Die Nadelabschnitte, welche bei der jeweiligen Nadel in die Nadelspitze münden, können für die Nadeln gleich oder verschieden ausgestaltet sein, insbesondere hinsichtlich der Formgebung in Bezug auf den Nadelquerschnitt. Auch kann die Relativlage des jeweiligen Nadelabschnitts mit der Nadelspitze in Bezug auf die Nadellängsachse gleich oder verschieden sein. Die Nadelabschnitte mit der jeweiligen Nadelspitze können zur Stechspitze hin schräg oder parallel zur Nadellängsachse verlaufen.

Zumindest ein Teil der mehreren Nadelspitzen kann entlang einer Stech- oder Einstichrichtung hintereinanderliegend angeordnet sein. Die Stech- oder Einstichrichtung kann im Wesentlichen parallel zur Nadellängsachse ausgebildet sein. Der zumindest eine Teil der mehreren Nadelspitzen, welcher entlang der Einstichrichtung hintereinander liegend angeordnet ist, liegt auf einer Stechlinie des Hautstechwerkzeugs.

Das Zuführreservoir kann zu einem Abstandsbereich zwischen den hintereinanderliegenden Nadelspitzen hin geöffnet sein.

Zumindest ein Teil der mehreren Nadelspitzen kann in einer Fläche quer zur Längsachse nebeneinanderliegend angeordnet sein. Die Nadelspitzen, welche in der Fläche quer zur Längsachse nebeneinander liegen, können voneinander beabstandet sein oder sich berühren.

Die mehreren Nadelspitzen können an der einen Nadel gebildet sein. Alternativ können die mehreren Nadelspitzen an mehreren Nadeln gebildet sein.

Jeweilige Längsachsen der mehreren Nadelspitzen können in einem von Null verschiedenen Winkel zueinander angeordnet sein. Die Längsachse der Nadelspitze entspricht der axialen Richtung in dem sich rückseitig unmittelbar an die Nadelspitze anschließenden Nadelabschnitt.

In einem Bereich der Nadel zwischen dem Nadelabschnitt und dem weiteren Nadelabschnitt kann ein Knick- oder Federabschnitt gebildet sein, derart, dass die Nadel bei Druckbelastung auf die zugeordnete Nadelspitze federnd einknickt. Aufgrund der federnden Knickbewegung kann das offene Reservoir momentan seine Form und / oder seine Größe verändern.

Zwischen dem Nadelabschnitt und dem weiteren Nadelabschnitt kann ein direkter Übergang gebildet sein. Der weitere Nadelabschnitt kann sich unmittelbar und direkt an den Nadelabschnitt anschließen. Der Übergang zwischen den beiden Nadelabschnitten kann zum Beispiel mittels des Knick- oder Federabschnitts gebildet sein. Der direkte Übergang kann als gebogener Übergang oder als eckiger Übergang ausgeführt sein.

Im Bereich des Zuführreservoirs kann dem Nadelabschnitt und / oder dem weiteren Nadelabschnitt gegenüberliegend ein gerader Nadelabschnitt angeordnet sein. Der gerade Nadelabschnitt kann Teil der das offene Reservoir bildenden und begrenzenden Nadelabschnitte sein. Nadelschäfte der mehreren Nadeln können einen gemeinsamen Werkzeugschaft bilden. Die Nadelschäfte können miteinander verdrillt und / oder verlötet sein. Die Nadelschäfte können an einer gemeinsamen Schaftaufnahme angeordnet sein, zum Beispiel zum Ausbilden einer Nadelplatte, bei der über eine Applikationsfläche mehrere Nadeln oder mehrere Hautstechwerkzeuge angeordnet sind. Die Nadelschäfte können voneinander beabstandet und in direktem Berührungskontakt stehen.

Eine oder mehrere seitliche Öffnungen des offenen Reservoirs können als ein jeweiliges Langloch gebildet sein.

Der weitere Nadelabschnitt, welcher zur Nadellängsachse hin geneigt oder gebogen ist, kann endseitig in eine Nadelspitze münden.

Die vorangehend in Verbindung mit dem Hautstechwerkzeug erläuterten Ausgestaltungen können für ein oder mehrere der Hautstechwerkzeuge an der Stechwerkzeuganordnung vorgesehen sein. Die mehreren Hautstechwerkzeuge können gleich oder verschieden ausgebildet sein. Die Stechspitzen der jeweiligen Hautstechwerkzeuge können bei der Stechwerkzeuganordnung eine gemeinsame Stechspitze bilden, wobei die Stechspitzen in einer gemeinsamen Ebene oder versetzt zueinander in hintereinander liegenden Ebenen angeordnet sein können.

### Beschreibung von Ausführungsbeispielen

Im Folgenden werden weitere Ausführungsformen unter Bezugnahme auf Figuren einer Zeichnung erläutert. Hierbei zeigen:
- Fig. 1: eine schematische Darstellung von mehreren Hautstechwerkzeugen, bei denen Nadelabschnitte gebogen sind,
- Fig. 2: eine schematische Darstellung von mehreren Haustechwerkzeugen, bei denen gerade Nadelabschnitte von einer Nadellängsachse weg und zur Nadellängsachse hin geneigt oder abgewinkelt sind,
- Fig. 3: eine schematische Darstellung von mehreren Hautstechwerkzeugen, bei denen Nadeln jeweils zur Nadellängsachse geneigte oder abgewinkelte Nadelabschnitte aufweisen, die gerade ausgebildet sind,
- Fig. 4: eine schematische Darstellung von Hautstechwerkzeugen gemäß einer weiteren Ausführungsform,
- Fig. 5: eine schematische Darstellung von weiteren Hautstechwerkzeugen,
- Fig. 6: eine schematische Darstellung von anderen Hautstechwerkzeugen,
- Fig. 7: eine schematische Darstellungen eines Hautstechwerkzeugs mit einer Flachnadel,
- Fig. 8: eine schematische Darstellung von Hautstechwerkzeugen mit jeweils mehr als zwei Nadeln,
- Fig. 9: eine schematische Darstellung einer Stechspitze eines Hautstechwerkzeugs, bei der Nadelspitzen hintereinanderliegend angeordnet sind, und
- Fig. 10: eine schematische Darstellung von Stechspitzen eines Hautstechwerkzeugs, bei der Nadelspitzen zusammenliegend angeordnet sind.

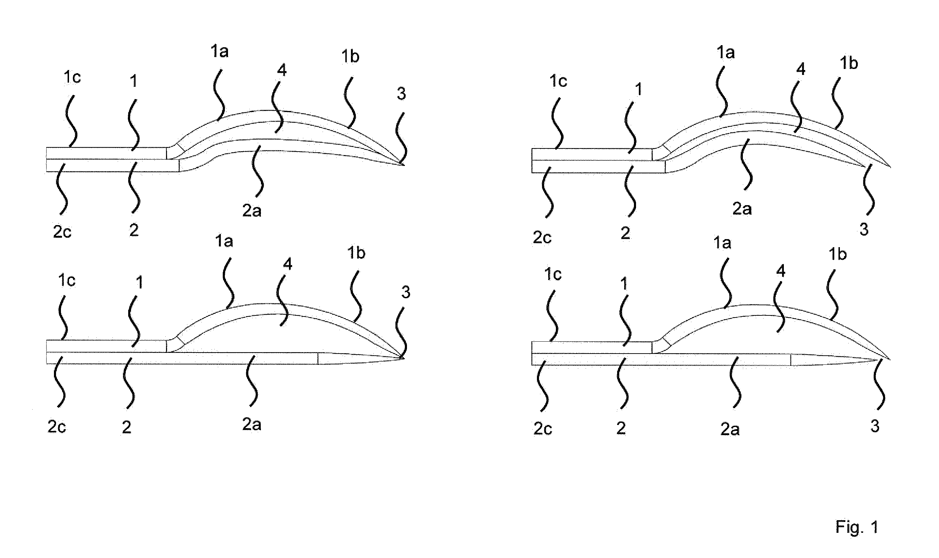

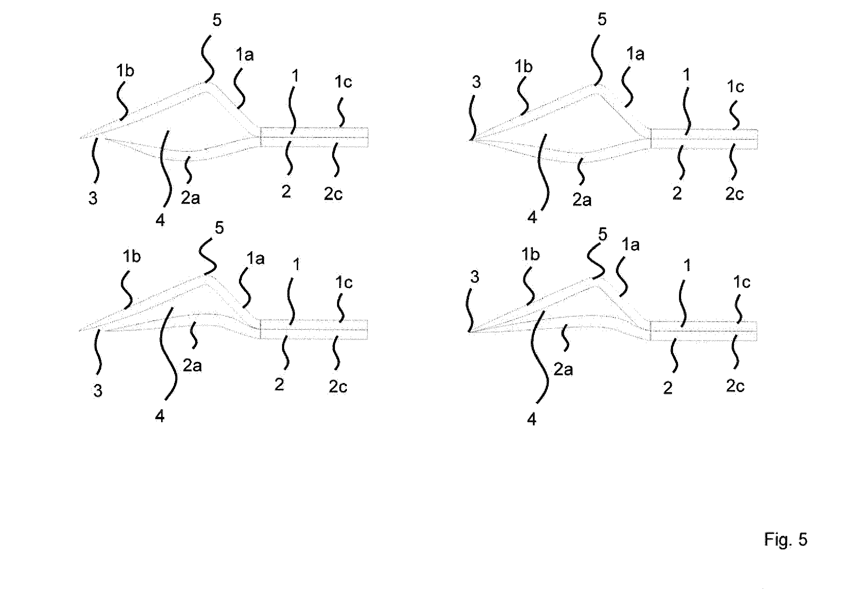

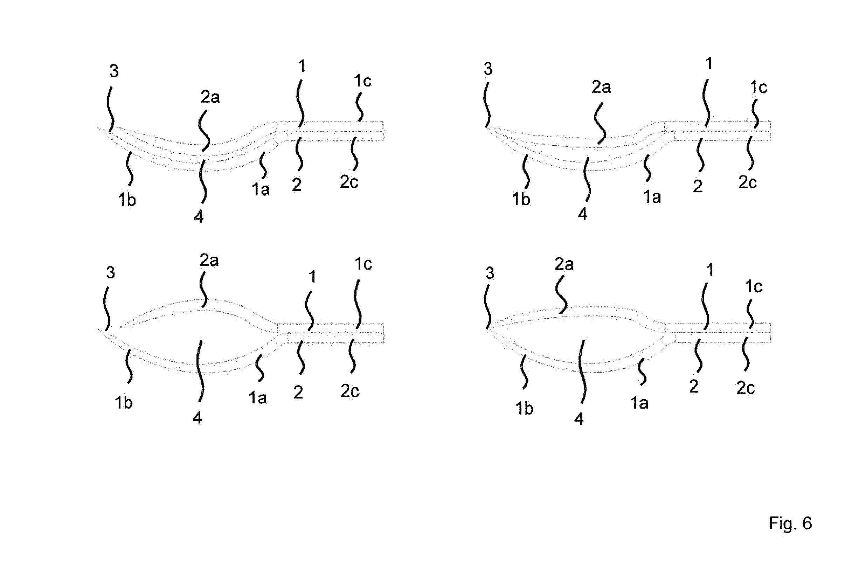

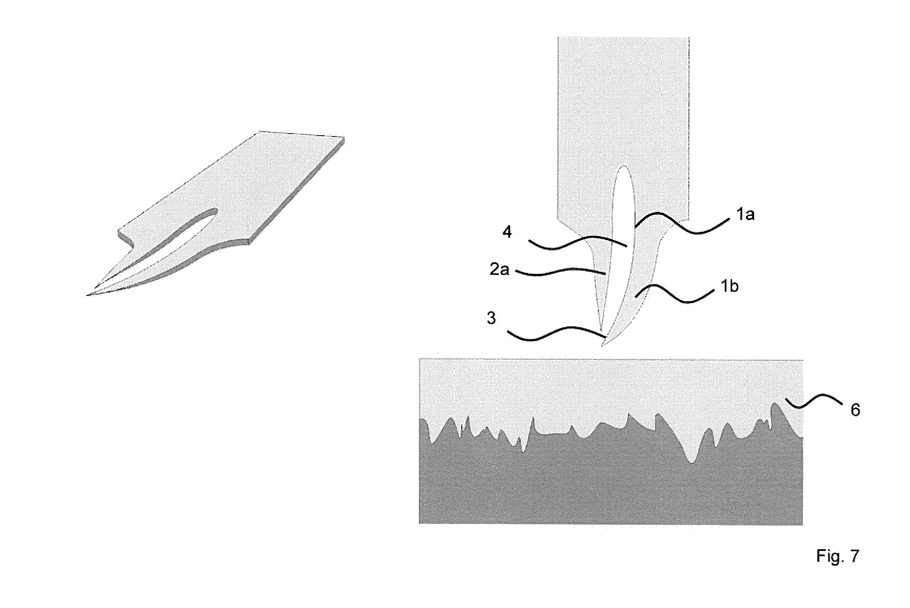

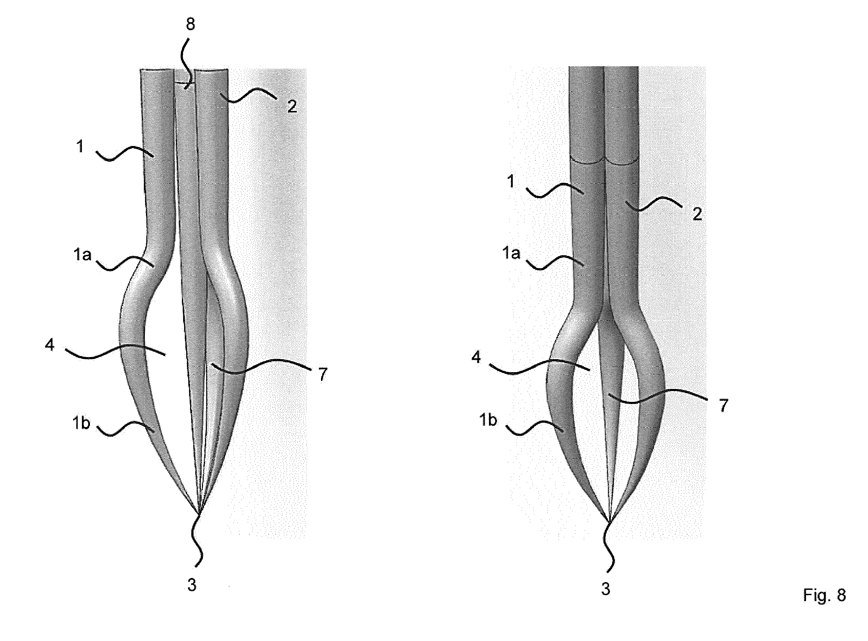

Unter Bezugnahme auf die Fig. 1 bis 8 werden nachfolgend Ausführungsformen für ein Hautstechwerkzeug zum lokalen Aufstechen einer menschlichen oder einer tierischen Haut erläutert. Bei den in den Fig. 1 bis 4 gezeigten Ausführungsformen weist das Hautstechwerkzeug jeweils eine erste und eine zweite Nadel 1, 2 auf. In den Fig. 5 und 6 sind weitere Ausgestaltungen von Hautstechwerkzeugen gezeigt. Bei der Ausführungsform in Fig. 7 ist eine Flachnadel gebildet. Fig. 8 zeigt Ausgestaltung eines Hautstechwerkzeugs mit mehr als zwei Nadeln.

Sämtliche Hautstechwerkzeuge verfügen über eine Stechspitze 3, die mit wenigstens zwei Nadelspitzen gebildet ist. Die beiden Nadelspitzen können hierbei in einer Stechrichtung hintereinanderliegend oder in einer gemeinsamen Stechspitze zusammenlaufend angeordnet sein. Die Stechrichtung kann parallel zur Längsachse des Hautstechwerkzeugs verlaufen.

Bei den in Fig. 1 gezeigten Ausführungen ist ein von der Nadellängsachse weg gerichteter oder geneigter Abschnitt 1a gebogen (gekrümmt). Vergleichbar ist ein sich hieran anschließender, wieder zur Nadellängsachse hin geneigter oder gerichteter Nadelabschnitt 1b gekrümmt oder gebogen. Ein gegenüberliegender Nadelabschnitt 2a der zweiten Nadel 2 ist gerade oder gekrümmt. Zwischen den Nadelabschnitten 1a, 1b der ersten Nadel 1 sowie dem Nadelabschnitt 2a der zweiten Nadel 2 ist ein Zuführreservoir 4 gebildet, welches als offenes Reservoir ausgeführt ist. In dem Zuführreservoir 4 kann sich eine in die Haut einzubringende Substanz sammeln, um dann im Betrieb zur Stechspitze 3 hin zu fließen. Nadelschäfte 1c, 2c der beiden Nadeln 1, 2 sind in der dargestellten Ausführungsform aneinanderliegend angeordnet.

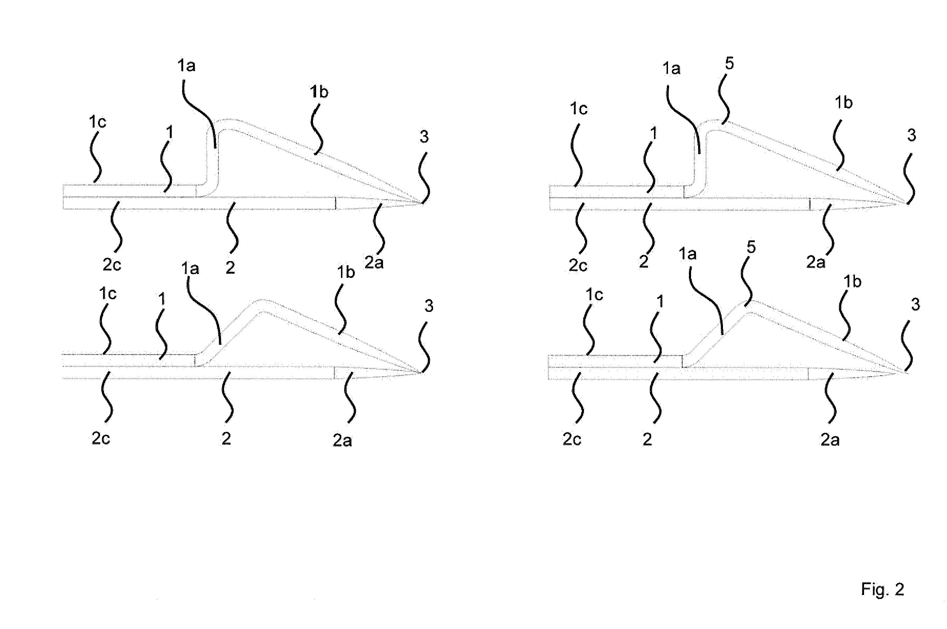

Bei den Ausführungsformen in Fig. 2 sind der von der Nadellängsachse weg geneigte oder gerichtete Nadelabschnitt 1a sowie der zur Nadellängsachse hin geneigte oder gerichtete Nadelabschnitt 1b als gerade Nadelabschnitte ausgeführt. Zwischen diesen Nadelabschnitten ist ein Eck- oder Knickbereich 5 angeordnet, über den beiden Nadelabschnitte 1a, 1b ineinander übergehen, insbesondere derart, dass die zugeordnete Nadel bei Druckbelastung auf die Stechspitze federnd einknickt.

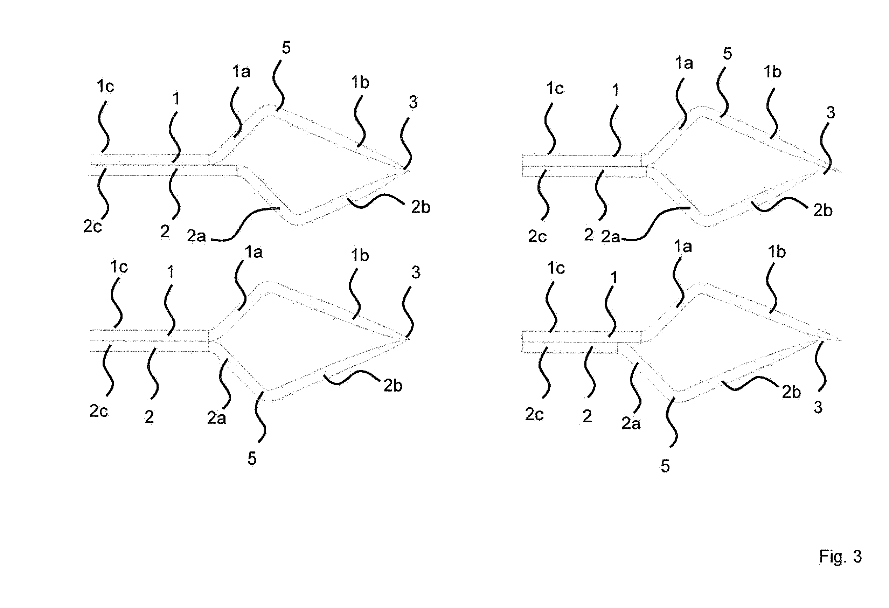

Vergleichbar weist das Hautstechwerkzeug bei den Ausgestaltungen in Fig. 3 für die zweite Nadel 2 gerade Nadelabschnitte 2a, 2b auf.

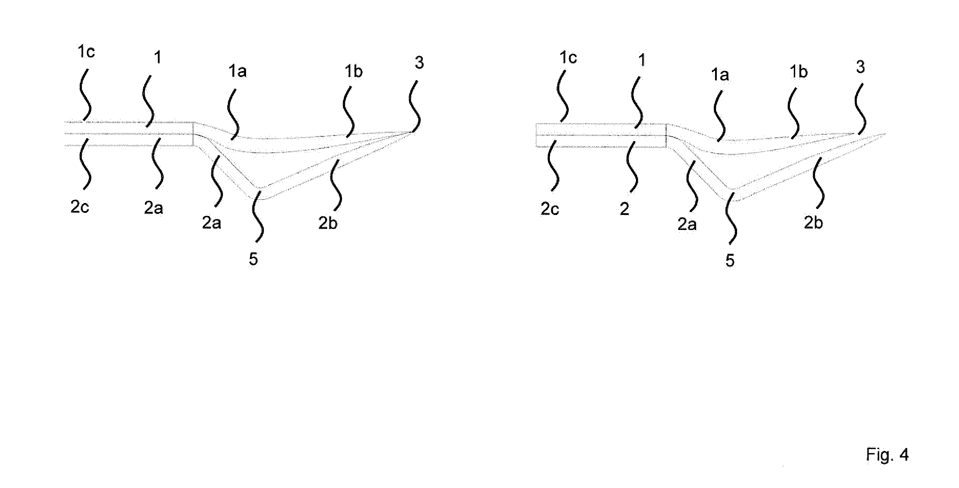

Bei dem Hautstechwerkzeug nach den Ausgestaltungen in Fig. 4 sind im Bereich des Zuführreservoirs 4 gekrümmte und gerade Nadelabschnitte kombiniert.

In den Fig. 5 und 6 sind weitere Ausgestaltungen von Hautstechwerkzeugen gezeigt.

Fig. 7 zeigt schematische Darstellungen einer Flachnadel mit dem Zuführreservoir 4, die Nadelspitzen sind an ein und derselben Flachnadel hergestellt, die zum Beispiel aus einem Blechmaterial hergestellt ist. Die Flachnadel ist in Fig. 7 oberhalb eines im Querschnitt dargestellten Hautabschnitts 6 gezeigt. Der von der Nadellängsachse weg gerichteter oder geneigter Abschnitt 1a ist benachbart zum Zuführreservoir 4 gebogen (gekrümmt) wie auch der sich hieran anschließende, wieder zur Nadellängsachse hin geneigte oder gerichtete Nadelabschnitt 1b gekrümmt oder gebogen ist. Der gegenüberliegende Nadelabschnitt 2a, welcher zumindest auf der dem Zuführreservoir 4 zugewandten Seite gerade oder gekrümmt ausgeführt sein kann, ist an derselben Flachnadel gebildet. Alternativ können ein oder beide von gegenüberliegenden und beabstandeten Nadelabschnitten, insbesondere im zur Stechspitze 3 distalen Bereich des Zuführreservoirs 4, auch im Wesentlichen parallel zur Nadellängsachse verlaufen.

Fig. 8 zeigt eine Ausführungsform eines Hautstechwerkzeugs mit insgesamt vier Nadeln 1, 2, 7, 8 sowie eine Ausführung mit drei Nadeln 1, 2, 7.

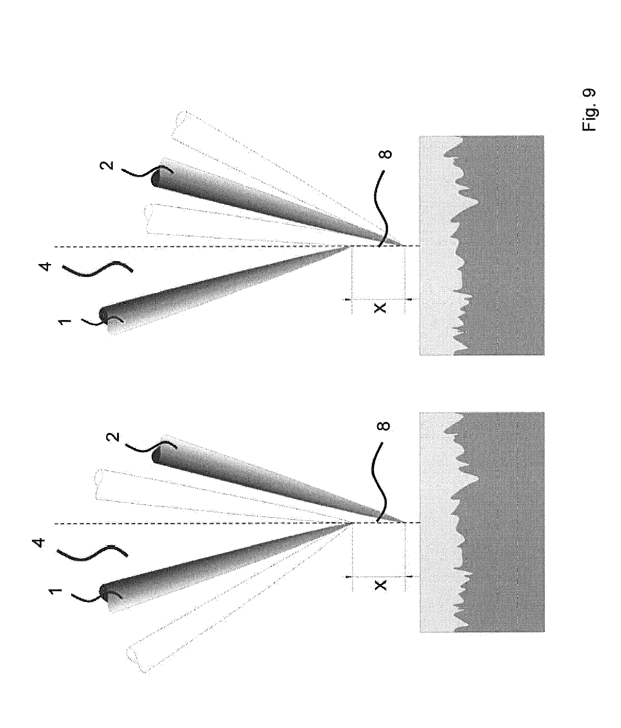

Fig. 9 zeigt schematische Darstellungen der Stechspitze 3 für verschiedene Ausgestaltungen, bei denen die Nadelspitzen hintereinanderliegend angeordnet sind. Das der Stechspitze 3 nachgelagerte Zuführreservoir 4 öffnet sich zu einem Abstandsbereich 8 zwischen den hintereinanderliegenden Nadelspitzen, welcher in Nadellängsrichtung eine Ausdehnung x aufweist.

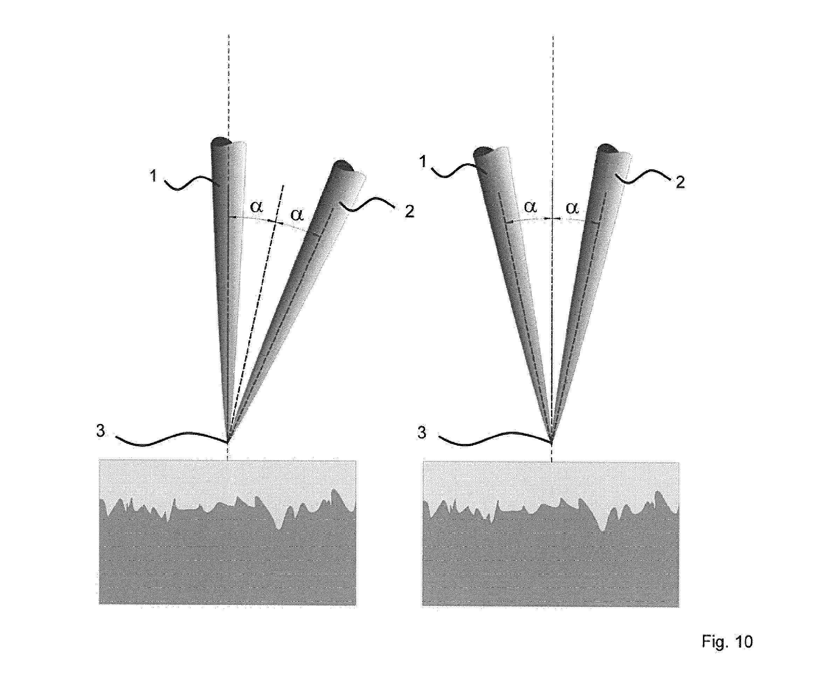

Im Unterschied hierzu sind die Nadelspitzen bei den Ausführungsformen der Stechspitze 3 in Fig. 10 zusammenliegend angeordnet.

Die in der vorstehenden Beschreibung, den Ansprüchen und der Zeichnung offenbarten Merkmale können sowohl einzeln als auch in beliebiger Kombination für die Verwirklichung der verschiedenen Ausführungen von Bedeutung sein.

## Patentansprüche

1. Hautstechwerkzeug zum lokalen Aufstechen einer menschlichen oder einer tierischen Haut, mit
- einem proximalen Werkzeugabschnitt,
- einem distalen Werkzeugabschnitt, in welchem eine Stechspitze angeordnet ist,
- einer oder mehreren Nadeln, bei denen ein Nadelschaft im Bereich des proximalen Werkzeugabschnitts und eine Nadelspitze als Teil der Stechspitze im Bereich des distalen Werkzeugabschnitts angeordnet sind, und
- einem Zuführreservoir, welches sich als offenes Reservoir zwischen einander gegenüberliegenden Nadelabschnitten der einen oder der mehreren Nadeln und in Richtung des proximalen Werkzeugabschnitts hinter der Stechspitze erstreckt, wobei das Zuführreservoir in Fließverbindung mit der Stechspitze steht, derart, dass im Betrieb eine einzutragende Substanz aus dem Zuführreservoir fließend zur Stechspitze hin gelangen kann,
wobei die einander gegenüberliegenden Nadelabschnitte an der einen Nadel oder an einer der mehreren Nadeln benachbart zu dem Zuführreservoir einen Nadelabschnitt, welcher von der Nadellängsachse weg geneigt oder gebogen ist, und einen weiteren Nadelabschnitt aufweisen, welcher zwischen dem Nadelabschnitt und der Nadelspitze angeordnet und zur Nadellängsachse hin geneigt oder gebogen ist.

2. Hautstechwerkzeug nach Anspruch 1, **dadurch gekennzeichnet, dass** die Stechspitze mit mehreren Nadelspitzen gebildet ist.

3. Hautstechwerkzeug nach Anspruch 2, **dadurch gekennzeichnet, dass** zumindest ein Teil der mehreren Nadelspitzen entlang einer Einstichrichtung hintereinanderliegend angeordnet sind.

4. Hautstechwerkzeug nach Anspruch 3, **dadurch gekennzeichnet, dass** das Zuführreservoir zu einem Abstandsbereich zwischen den hintereinanderliegenden Nadelspitzen hin geöffnet ist.

5. Hautstechwerkzeug nach mindestens einem der Ansprüche 2 bis 4, **dadurch gekennzeichnet, dass** zumindest ein Teil der mehreren Nadelspitzen in einer Fläche quer zur Längsachse nebeneinanderliegend angeordnet sind.

6. Hautstechwerkzeug nach mindestens einem der Ansprüche 2 bis 5, **dadurch gekennzeichnet, dass** die mehreren Nadelspitzen an der einen Nadel gebildet sind.

7. Hautstechwerkzeug nach mindestens einem der Ansprüche 2 bis 5, **dadurch gekennzeichnet, dass** jeweilige Längsachsen der mehreren Nadelspitzen in einem von Null verschiedenen Winkel zueinander angeordnet sind.

8. Hautstechwerkzeug nach mindestens einem der vorangehenden Ansprüche, **dadurch gekennzeichnet, dass** in einem Bereich der Nadel zwischen dem Nadelabschnitt und dem weiteren Nadelabschnitt ein Knick- oder Federabschnitt gebildet ist, derart, dass die Nadel bei Druckbelastung auf die zugeordnete Nadelspitze federnd einknickt.

9. Hautstechwerkzeug nach mindestens einem der vorangehenden Ansprüche, **dadurch gekennzeichnet, dass** zwischen dem Nadelabschnitt und dem weiteren Nadelabschnitt ein direkter Übergang gebildet ist.

10. Hautstechwerkzeug nach mindestens einem der vorangehenden Ansprüche, **dadurch gekennzeichnet, dass** im Bereich des Zuführreservoirs dem Nadelabschnitt und / oder dem weiteren Nadelabschnitt gegenüberliegend ein gerader Nadelabschnitt angeordnet ist.

11. Hautstechwerkzeug nach mindestens einem der vorangehenden Ansprüche, dadurch **ge- kennzeichnet,** dass Nadelschäfte der mehreren Nadeln einen gemeinsamen Werkzeugschaft bilden.

12. Hautstechwerkzeug nach mindestens einem der vorangehenden Ansprüche, **dadurch gekennzeichnet, dass** eine oder mehrere seitliche Öffnungen des offenen Reservoirs als ein jeweiliges Langloch gebildet sind.

13. Hautstechwerkzeug nach mindestens einem der vorangehenden Ansprüche, dadurch g e - **kennzeichnet,** dass der weitere Nadelabschnitt, welcher zur Nadellängsachse hin geneigt oder gebogen ist, endseitig in eine Nadelspitze mündet.

14. Stechwerkzeuganordnung mit mehreren Hautstechwerkzeugen zum lokalen Aufstechen einer menschlichen oder einer tierischen Haut nach mindestens einem der vorangehenden Ansprüche, wobei die mehreren Hautstechwerkzeuge an einer gemeinsamen Werkzeugaufnahme angeordnet sind.

## Geänderte Patentansprüche

### Geänderte Patentansprüche gemäss Regel 137(2) EPÜ.

1. Hautstechwerkzeug zum lokalen Aufstechen einer menschlichen oder einer tierischen Haut, mit
- einem proximalen Werkzeugabschnitt,
- einem distalen Werkzeugabschnitt, in welchem eine Stechspitze (3) angeordnet ist,
- einer oder mehreren Nadeln (1; 2), bei denen ein Nadelschaft im Bereich des proximalen Werkzeugabschnitts und eine Nadelspitze als Teil der Stechspitze (4) im Bereich des distalen Werkzeugabschnitts angeordnet sind, und
- einem Zuführreservoir (4), welches sich als offenes Reservoir zwischen einander gegenüberliegenden Nadelabschnitten der einen oder der mehreren Nadeln (1; 2) und in Richtung des proximalen Werkzeugabschnitts hinter der Stechspitze (3) erstreckt, wobei das Zuführreservoir (4) in Fließverbindung mit der Stechspitze (3) steht, derart, dass im Betrieb eine einzutragende Substanz aus dem Zuführreservoir (4) fließend zur Stechspitze (3) hin gelangen kann,
wobei die einander gegenüberliegenden Nadelabschnitte an der einen Nadel oder an einer der mehreren Nadeln (1; 2) benachbart zu dem Zuführreservoir (4) einen Nadelabschnitt (1b), welcher zur Nadellängsachse hin geneigt oder gebogen ist, aufweisen, **dadurch gekennzeichnet, dass** die einander gegenüberliegenden Nadelabschnitte an der einen Nadel oder an einer der mehreren Nadeln (1; 2) benachbart zu dem Zuführreservoir (4) einen weiteren Nadelabschnitt (1a) aufweisen, wobei der Nadelabschnitt (1b) zwischen dem weiteren Nadelabschnitt (1a) und der Nadelspitze angeordnet und von der Nadellängsachse weg geneigt oder gebogen ist.

2. Hautstechwerkzeug nach Anspruch 1, **dadurch gekennzeichnet, dass** die Stechspitze (3) mit mehreren Nadelspitzen gebildet ist.

3. Hautstechwerkzeug nach Anspruch 2, **dadurch gekennzeichnet, dass** zumindest ein Teil der mehreren Nadelspitzen entlang einer Einstichrichtung hintereinanderliegend angeordnet sind.

4. Hautstechwerkzeug nach Anspruch 3, **dadurch gekennzeichnet, dass** das Zuführreservoir zu einem Abstandsbereich zwischen den hintereinanderliegenden Nadelspitzen hin geöffnet ist.

5. Hautstechwerkzeug nach mindestens einem der Ansprüche 2 bis 4, **dadurch gekennzeichnet, dass** zumindest ein Teil der mehreren Nadelspitzen in einer Fläche quer zur Längsachse nebeneinanderliegend angeordnet sind.

6. Hautstechwerkzeug nach mindestens einem der Ansprüche 2 bis 5, **dadurch gekennzeichnet, dass** die mehreren Nadelspitzen an der einen Nadel gebildet sind.

7. Hautstechwerkzeug nach mindestens einem der Ansprüche 2 bis 5, **dadurch gekennzeichnet, dass** jeweilige Längsachsen der mehreren Nadelspitzen in einem von Null verschiedenen Winkel zueinander angeordnet sind.

8. Hautstechwerkzeug nach mindestens einem der vorangehenden Ansprüche, **dadurch gekennzeichnet, dass** in einem Bereich der Nadel zwischen dem Nadelabschnitt (1b) und dem weiteren Nadelabschnitt (1a) ein Knick- oder Federabschnitt (5) gebildet ist, derart, dass die Nadel bei Druckbelastung auf die zugeordnete Nadelspitze federnd einknickt.

9. Hautstechwerkzeug nach mindestens einem der vorangehenden Ansprüche, **dadurch gekennzeichnet, dass** zwischen dem Nadelabschnitt (1b) und dem weiteren Nadelabschnitt (1a) ein direkter Übergang gebildet ist.

10. Hautstechwerkzeug nach mindestens einem der vorangehenden Ansprüche, **dadurch gekennzeichnet, dass** im Bereich des Zuführreservoirs (4) dem Nadelabschnitt (1b) und / oder dem weiteren Nadelabschnitt (1a) gegenüberliegend ein gerader Nadelabschnitt angeordnet ist.

11. Hautstechwerkzeug nach mindestens einem der vorangehenden Ansprüche, **dadurch gekennzeichnet, dass** Nadelschäfte der mehreren Nadeln (1, 2) einen gemeinsamen Werkzeugschaft bilden.

12. Hautstechwerkzeug nach mindestens einem der vorangehenden Ansprüche, **dadurch gekennzeichnet, dass** eine oder mehrere seitliche Öffnungen des offenen Reservoirs als ein jeweiliges Langloch gebildet sind.

13. Hautstechwerkzeug nach mindestens einem der vorangehenden Ansprüche, **dadurch gekennzeichnet, dass** der weitere Nadelabschnitt (1a), welcher zur Nadellängsachse hin geneigt oder gebogen ist, endseitig in eine Nadelspitze mündet.

14. Stechwerkzeuganordnung mit mehreren Hautstechwerkzeugen zum lokalen Aufstechen einer menschlichen oder einer tierischen Haut nach mindestens einem der vorangehenden Ansprüche, wobei die mehreren Hautstechwerkzeuge an einer gemeinsamen Werkzeugaufnahme angeordnet sind.
